# EUROPÄISCHE PATENTSCHRIFT

(11) **EP 1 492 476 B1**
(45) Veröffentlichungstag und Bekanntmachung des Hinweises auf die Patenterteilung: **19.07.2006**
(21) Anmeldenummer: 02708130.6
(22) Anmeldetag: 11.04.2002
(51) Int. Cl.: A61F 2/46, B01F 13/00

(54) **VORRICHTUNG ZUR MISCHUNG UND/ODER INJEKTION VON ZEMENTEN**
DEVICE FOR MIXING AND/OR INJECTING CEMENTS
DISPOSITIF DE MELANGE ET/OU D'INJECTION DE CIMENTS

(43) Veröffentlichungstag der Anmeldung: 05.01.2005
(73) Patentinhaber: Synthes AG Chur, 7002 Chur (CH)
(72) Erfinder: FREI, Christian, CH-4053 Basel (CH); EHRSAM, Beat, CH-4436 Oberdorf (CH)
(74) Vertreter: Lusuardi, Werther
(86) Internationale Anmeldenummer: PCT/CH2002/000204
(87) Internationale Veröffentlichungsnummer: WO 2003/084445

(56) Entgegenhaltungen:
- EP-A- 0 987 055
- DE-U- 29 721 534
- FR-A- 2 119 650
- US-A- 3 417 971
- US-A- 4 010 934
- US-A- 4 758 096

## Beschreibung

Die Erfindung bezieht sich auf eine Vorrichtung zur Lagerung und/oder Mischung und/oder Injektion von Zementen, insbesondere von Knochenzementen gemäss dem Oberbegriff des Patentanspruchs 1. Eine solche Vorrichtung ist zum Beispiel aus US-A-4758096 bekannt.

Viele der heutigen Zemente, welche aus zwei Komponenten, einer pulverförmigen Komponente und einer flüssigen Komponente hergestellt werden, polymerisieren nach einer gründlichen Mischung der Komponenten und bilden in der Folge eine harte und mehr oder weniger dauerhafte Zementmasse. Neben den polymerisierenden Zementen werden auch sogenannte hydraulische Zemente, beispielsweise Phosphatzemente eingesetzt.

Die vorliegende Erfindung ist für verschiedene solche Zemente einsetzbar. Insbesondere ist sie auch für die Präparierung von sogenannten Knochenzementen geeignet, welche zur Verankerung und Unterstützung von künstlichen Gelenkkomponenten und anderen Prothesen einsetzbar sind.

Der gegenwärtig meist eingesetzte Knochenzement ist Polymethylmethacrylate oder auch PMMA genannt. PMMA umfasst ein pulverförmiges Polymer und ein flüssiges Monomer. Nach dem Mischen polymerisieren diese Komponenten innerhalb von Minuten und bilden eine feste Verbindung zwischen der Prothese und der die Prothese umgebenden Knochenstruktur.

Zusätzlich kommen heute vermehrt auch Calzium Phosphat Zemente zum Einsatz, wo ebenfalls eine pulverförmige Komponente mit einer flüssigen Komponente vermischt wird. Nach dem Mischen der Komponenten kommt es durch eine Ausfällungsreaktion zum Aushärten des Zementes.

Eine Vorrichtung zur Mischung und Dosierung von pharmazeutischen Produkten ist aus der DE 297 21 534 WEPA bekannt. Diese bekannte Vorrichtung umfasst im wesentlichen einen Misch- und Dosierbehälter mit einem Behälterkörper, welcher einen zylindrischen Innenraum und einen im Durchmesser verjüngten Auslass am einen Ende umfasst, eine Kolbeneinheit, welche am anderen Ende des Behälterkörpers dichtend und axial bewegbar in den Innenraum eingesetzt werden kann, und eine Antriebseinrichtung. Die Kolbeneinheit weist einen Durchbruch für eine Antriebswelle eines im Innenraum antreibbaren Mischwerkzeuges auf. Die Antriebseinrichtung greift in den Durchbruch ein und ist mit dem Behälterkörper verbindbar, wobei die Antriebseinrichtung mit dem Behälterkörper erst nach dem Entfernen der Antriebswelle für das Mischwerkzeug verbindbar ist. Die Kolbeneinheit wird mittels einer Gewindestange in Richtung auf den Auslass bewegt, wobei die Gewindestange durch eine manuelle Betätigung eines Betätigungselementes an der Antriebseinrichtung angetrieben wird. Nachteilig an dieser bekannten Vorrichtung ist, dass zwischen dem Mischwerkzeug und der Mischraumwand nicht zwangsläufig ein ausreichender Abstand gewährleistet wird, so dass beim Mischen von abrasiven Pulvern ein erheblicher Abrieb am Mischwerkzeug und/oder der Mischraumwand auftreten kann, wobei die abgetragenen Teilchen die Qualität des Produktes negativ beeinflussen können.

Hier will die Erfindung Abhilfe schaffen. Der Erfindung liegt die Aufgabe zugrunde, eine Vorrichtung zum Mischen eines Zweikomponenten-Zementes zu schaffen, welche Arretiermittel für das Mischwerkzeug umfasst, wodurch ein Minimalabstand zwischen Mischwerkzeug und Mischraumwand gewährleistet wird und daher durch diese Arrtetiermittel auch bei abrasiven Pulvern einen Abrieb am Mischwerkzeug und/oder der Mischraumwand vermieden wird.

Die Erfindung löst die gestellte Aufgabe mit einer Vorrichtung zur Lagerung und/oder Mischung und/oder Injektion von Zementen, insbesondere von Knochenzementen, welche die Merkmale des Anspruchs 1 aufweist.

Die erfindungsgemässe Vorrichtung umfasst im wesentlichen einen Tubus mit einer Austrittsöffnung am vorderen Ende, einem am hinteren Ende des Tubus mittels erster Verriegelungsmittel lösbar befestigbaren Deckel mit einer zur Längsachse des Tubus koaxialen Bohrung und einen im Hohlraum axial und rotativ bewegbaren Mischer mit einem Mischwerkzeug und einem die Bohrung im Deckel durchdringenden Mischerschaft. Der Mischerschaft ist ausserhalb des Tubus mit Antriebsmitteln zur axialen und rotativen Bewegung des Mischers verbindbar. Ein Verschluss ist lösbar mit dem vorderen Ende des Tubus verbindbar. Dieser Verschluss umfasst eine axial durchgehende Zentralbohrung, welche mit der Austriffsöffnung des Tubus verbunden und durch eine Membran verschlossen ist. Durch die Membran wird die Austrittsöffnung einerseits verschlossen und andererseits ist ohne Öffnen der Vorrichtung die flüssige Komponente des Zementes einspritzbar. Ferner umfasst die Vorrichtung Arretiermittel, durch welche die zur Längsachse parallele Verschiebung des Mischwerkzeuges mindestens gegen das vordere Ende des Tubus begrenzbar ist.

In der bevorzugten Ausführungsform der erfindungsgemässen Vorrichtung umfasst diese einen in den Hohlraum des Tubus vom hinteren Ende des Tubus einführbaren und axial im Hohlraum verschiebbaren Kolben mit einer zur Längsachse koaxialen Durchgangsbohrung und einer zur Längsachse konzentrischen Dichtlippe, wodurch der Hohlraum gegen das hintere Ende des Tubus abdichtbar ist. Der Mischerschaft wird durch die Durchgangsbohrung im Kolben gelagert. Ferner ist der Tubus an seinem hinteren Ende lösbar mit einem Deckel verschliessbar, wobei der Deckel koaxial zur Längsachse des Hohlraumes durchbohrt ist und der Mischerschaft ebenfalls durch diese Bohrung durchführbar ist. Ferner sind die axialen Arretiermittel so ausgestaltet, dass die Antriebsmittel, wenn sich das Mischwerkzeug am vorderen Ende des Tubus in seiner vordersten Position befindet, auf der bezüglich des Hohlraumes betrachteten Aussenseite des Deckels so zur Anlage kommen, dass zwischen dem Mischwerkzeug und der am vorderen Ende des Tubus den Hohlraum axial begrenzenden, vorderen Hohlraumwand einen Minimalabstand A verbleibt. Dieser Minimalabstand A beträgt vorzugsweise zwischen dem zwei- bis fünffachen Durchmesser der grössten Partikel im Mischgut.

Bei Knochenzementen kann der Durchmesser dieser Partikel innerhalb der folgenden Bereiche liegen:
- zwischen 5µm und 50 µm;
- zwischen 50 µm und 800 µm; oder aber auch
- zwischen 5 µm und 800 µm.

In verschiedenen Ausführungsformen der erfindungsgemässen Vorrichtung beträgt der Minimalabstand A zwischen:
- 10 µm und 250 µm; oder zwischen
- 100 µm und 4000 µm; oder zwischen
- 10 µm und 4000 µm.

Vorzugsweise ist der Hohlraum koaxial kreiszylindrisch ausgestaltet und weist senkrecht zur Längsachse betrachtet einen Durchmesser D auf, welcher grösser ist als die senkrecht zur Längsachse betrachtete maximale Abmessung X des Mischwerkzeuges, wobei die Differenz Δ = D - X vorzugsweise zwischen dem vier- bis zehnfachen Durchmesser der grössten Partikel im Mischgut beträgt.

In verschiedenen Ausführungsformen der erfindungsgemässen Vorrichtung beträgt diese Differenz Δ zwischen:
- 20 µm und 500 µm; oder zwischen
- 200 µm und 8000 µm; oder zwischen
- 20 µm und 8000 µm.

Die Durchmesser des Mischerschaftes und der Durchgangsbohrung im Kolben sind so ausgestaltet, dass der Mischerschaft in der Durchgangsbohrung ein geringes Spiel aufweist, so dass der Kolben während des Mischvorganges durch den Mischer nicht bewegt wird.

In einer anderen Ausführungsform der erfindungsgemässen Vorrichtung umfasst diese zweite Arretiermittel, welche derart ausgestaltet sind, dass die axiale Verschiebung des Mischwerkzeuges auch gegen das hintere Ende des Tubus begrenzt wird. Die axiale Begrenzung ist derart bemessen, dass das Mischwerkzeug, wenn es sich am hinteren Ende des Tubus in seiner hintersten Position befindet, relativ zur vorderen Stirnfläche des ebenfalls in seine hinterste Position verschobenen Kolbens einen Minimalabstand B aufweist. Vorzugsweise entspricht dieser Minimalabstand B dem Minimalabstand A.

Durch den Abstand des Mischwerkzeuges von der vorderen Hohlraumwand, der seitlichen Hohlraumwand des Tubus sowie von der vorderen Stirnfläche des Kolbens wird gewährleistet, dass beim Mischen von abrasiven Teilchen kein oder nur ein geringer Abrieb an den Hohlraumwänden oder am Kolben entsteht. Dabei soll der Abstand vorzugsweise mehr als das Doppelte der Partikeldurchmesser betragen.

In einer weiteren Ausführungsform der erfindungsgemässen Vorrichtung sind die ersten Arretiermittel durch einen ersten, an den Antriebsmitteln angebrachten, axialen Anschlag realisiert, wobei dieser erste axiale Anschlag vorzugsweise so ausgeführt ist, dass die Antriebsmittel so ausgestaltet sind, dass sie am Deckel zur Anlage kommen, wenn das Mischwerkzeug in seiner vordersten Position ist.

In wiederum einer weiteren Ausführungsform der erfindungsgemässen Vorrichtung sind die zweiten Arretiermittel durch einen zweiten am Mischerschaft und am Deckel angebrachten, axialen Anschlag realisiert, wobei dieser zweite axiale Anschlag vorzugsweise so ausgeführt ist, dass die zur Längsachse orthogonale Querschnittsfläche des Mischerschaftes in einem Abstand L₁ vom vorderen Ende des Mischwerkzeuges verkleinert ist und dazu komplementär in einem Abstand L₂ von der Deckelinnenfläche die ebenfalls zur Längsachse orthogonale Querschnittsfläche der Bohrung im Deckel verkleinert ist. Die Abstände L₁ und L₂ sind abhängig von der axialen Dicke des Mischwerkzeuges, dem Mininalabstand B und der Kolbenlänge.

Die ersten Verriegelungsmittel, mittels welcher der Deckel lösbar mit dem hinteren Ende des Tubus verbindbar ist, können in verschiedenen Ausführungsformen der erfindungsgemässen Vorrichtung als Bajonettverschluss, als Schraubverbindung oder auch als Schnappverschluss ausgestaltet sein.

Analog zu den ersten Verriegelungsmitteln können die zweiten Verriegelungsmittel, mittels welcher der Verschluss am vorderen Ende des Tubus lösbar befestigbar ist, in verschiedenen Ausführungsformen der erfindungsgemässen Vorrichtung als Bajonettverschluss, als Schraubverbindung oder auch als Schnappverschluss ausgestaltet sein.

Die Antriebsmittel zur axialen und/oder rotativen Bewegung des Mischers bestehen vorzugsweise aus einem Handgriff, können aber in anderen Ausführungsformen der erfindungsgemässen Vorrichtung auch durch eine elektrisch, pneumatisch oder hydraulisch arbeitende Antriebsmaschine ausgeführt sein.

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass dank der erfindungsgemässen Vorrichtung wegen der Beabstandung zwischen Mischwerkzeug und Mischbehälter kein vom Mischbehälter oder Mischwerkzeug stammendes Material in das Mischgut gelangt. Dies ist für Implantatwerkstoffe äusserst wichtig, da Partikel bestimmter Materialien zu unerwünschten Wirkungen im Körper führen können. Zudem kann in der erfindungsgemässen Vorrichtung die pulverförmige Komponente des Zementes gelagert und zum gewünschten Zeitpunkt mit der flüssigen Komponente gemischt werden. Anschliessend kann die Mischung direkt aus dem Mischraum an den gewünschten Ort injiziert werden. Der Vorteil besteht darin, dass damit das Mischgut immer von der Umgebung und möglichen Verunreinigungen geschützt ist.

Die Erfindung und Weiterbildungen der Erfindung werden im folgenden anhand der teilweise schematischen Darstellungen mehrerer Ausführungsbeispiele noch näher erläutert.

### Es zeigen:

Fig. 1 einen Längsschnitt durch eine Ausführungsform der erfindungsgemässen Vorrichtung, wobei der Mischer in seiner vordersten Position ist; und

Fig. 2 einen Längsschnitt durch die in Fig. 1 dargestellte Ausführungsform der erfindungsgemässen Vorrichtung, wobei der Mischer in seiner hintersten Position ist.

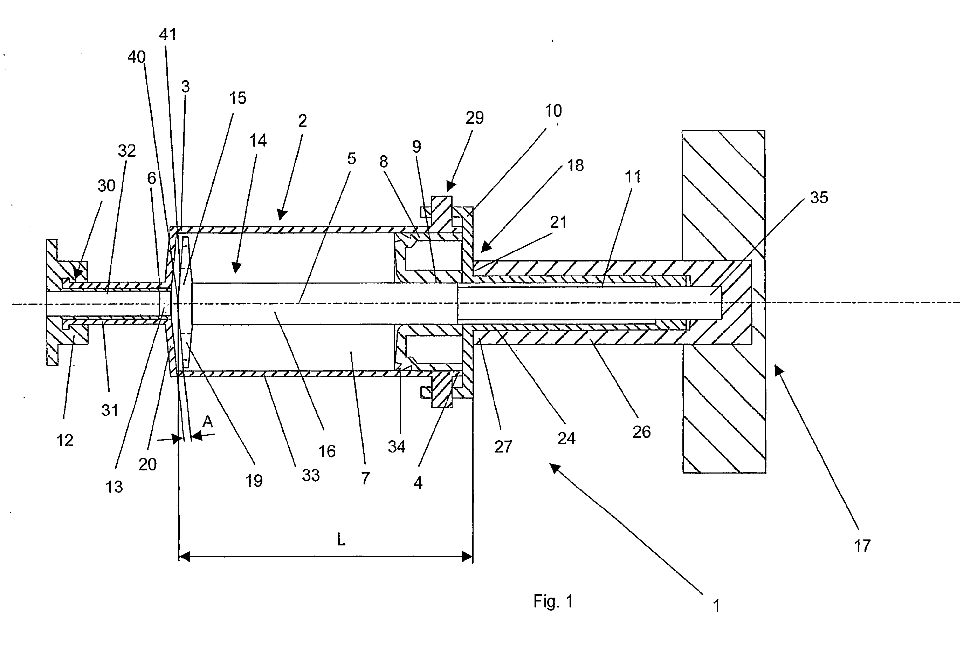

Fig. 1 zeigt eine Ausführungsform der erfindungsgemässen Vorrichtung 1 mit einem Tubus 2 und einem axial in der vordersten Position stehenden Mischer 14, welcher im Hohlraum 7 des Tubus 2 parallel zur Längsachse 5 verschiebbar und um die Längsachse 5 rotierbar aufnehmbar ist. Der Tubus 2 ist konzentrisch zur Längsachse 5 hohlzylindrisch ausgestaltet, umfasst quer zur Längsachse 5 gerichtet eine vordere Hohlraumwand 20 sowie parallel zur Längsachse 5 eine seitliche Hohlraumwand 33 und weist am vorderen Ende 3 eine Austrittsöffnung 6 auf, deren zur Längsachse 5 orthogonale Querschnittsfläche q kleiner als die zur Längsachse 5 orthogonale Querschnittsfläche Q des Hohlraumes 7 im Tubus 2 ist. Am hinteren Ende 4 umfasst der Tubus 2 an seiner Aussenwand angeordnete, erste Verriegelungsmittel 29, welche dazu dienen, den Deckel 10 am hinteren Ende 4 des Tubus 2 lösbar zu befestigen. In der hier dargestellten, bevorzugten Ausführungsform der erfindungsgemässen Vorrichtung 1 sind diese ersten Verriegelungsmittel 29 als Bajonettverschluss ausgestaltet. Am vorderen Ende 3 des Tubus 2 ist koaxial zur Längsachse 5 ein hülsenförmiger Ansatz 31 angeordnet, welcher koaxial zur Längsachse 5 und zur Austrittsöffnung 6 fluchtend durchbohrt ist. Am Ansatz 31 sind zweite Verriegelungsmittel 30 angeordnet, so dass ein Verschluss 12 am Ansatz 31 lösbar befestigbar ist, wodurch der Hohlraum 7 am vorderen Ende 3 des Tubus 2 verschliessbar ist. Der Verschluss 12 ist koaxial zur Längsachse 5 und bis zur Austrittsöffnung 6 in den Ansatz 31 einschiebbar und weist eine zur Längsachse 5 koaxiale Zentralbohrung 32 auf, welche mittels einer Membran 13 verschlossen ist. Der Kolben 8 ist im Hohlraum 7 des Tubus 2 parallel zur Längsachse 5 verschiebbar und dient zur Dosierung des Mischgutes, welches bei einer Bewegung des Kolbens 8 parallel zur Längsachse 5 gegen das vordere Ende 3 des Tubus 2 aus der Austrittsöffnung 6 austritt. Ferner ist der Kolben 8 mit einer zur Längsachse 5 koaxialen Durchgangsbohrung 9 ausgestattet, welche zur Aufnahme und Führung des Mischerschaftes 16 dient. In Fig. 1 ist der Kolben 8 in seiner hinteren Stellung am hinteren Ende 4 des Tubus 2. Der Kolben 8 umfasst eine konzentrisch zur Längsachse 5 angeordnete Dichtlippe 34, wodurch der Hohlraum 7 gegen das hintere Ende 4 des Tubus 2 abdichtbar ist. Der Deckel 10, welcher lösbar koaxial zur Längsachse 5 am hinteren Ende 4 des Tubus 2 anbringbar ist, ist zur Längsachse 5 koaxial mit einer Bohrung 11 durchbohrt. Ferner umfasst der Deckel 10 eine zur Längsachse 5 koaxiale Hülse 24, welche ebenfalls koaxial von der Bohrung 11 durchdrungen wird, so dass der Mischerschaft 16 axial durch den Deckel 10 und die Hülse 24 durchführbar ist. Dem Mischwerkzeug 15 axial entgegengesetzt angeordnet sind am hinteren Ende 35 des Mischerschaftes 16 die Antriebsmittel 17 für den Mischer 14, welcher hier als Handgriff ausgestaltet sind. Der Mischer 14 ist im Hohlraum 7 axial und rotativ bewegbar und umfasst ein Mischwerkzeug 15 und einen durch die Durchgangsbohrung 9 im Kolben 8 und die Bohrung 11 im Deckel 10 und in der Hülse 24 durchführbaren Mischerschaft 16. Das Mischwerkzeug 15 ist mit parallel zur Längsachse 5 angeordneten, das Mischwerkzeug 15 axial durchdringenden Durchgangsöffnungen 19 ausgestattet. Ferner umfasst die Vorrichtung 1 erste Arretiermittel 18, welche die axiale Verschiebbarkeit des Mischers 14 gegen das vordere Ende 3 des Tubus 2 derart begrenzen, dass wenn das Mischwerkzeug 15 in seiner vordersten Position ist, zwischen der vorderen Stirnwand 41 des Mischwerkzeuges 15 und der vorderen Hohlraumwand 20 ein Minimalabstand A verbleibt. Die ersten Arretiermittel 18 bestehen in dieser Ausführungsform der erfindungsgemässen Vorrichtung 1 aus einem ersten axialen Anschlag 21, bei welchem das vordere Ende 27 des hülsenförmigen Fortsatzes 26 an den Antriebsmitteln 17 an der bezüglich des Tubus 2 äusseren, orthogonal zur Längsachse 5 gerichteten Oberfläche des Deckels 10 anliegt. Damit der Minimalabstand A eingehalten wird, ist der Mischerschaft 16 so mit den Antriebsmitteln 17 verbunden, dass zwischen dem vorderen, gegen die Austrittsöffnung 6 gerichteten Ende 40 des Mischwerkzeuges 15 und dem vorderen Ende 27 des hülsenförmigen Fortsatzes 26 an den Antriebsmitteln 17 eine Länge L eingehalten ist, welche durch die Länge des Hohlraumes 7 im Tubus 2, die Dicke des Deckels 10 und den Minimalabstand A bestimmt ist.

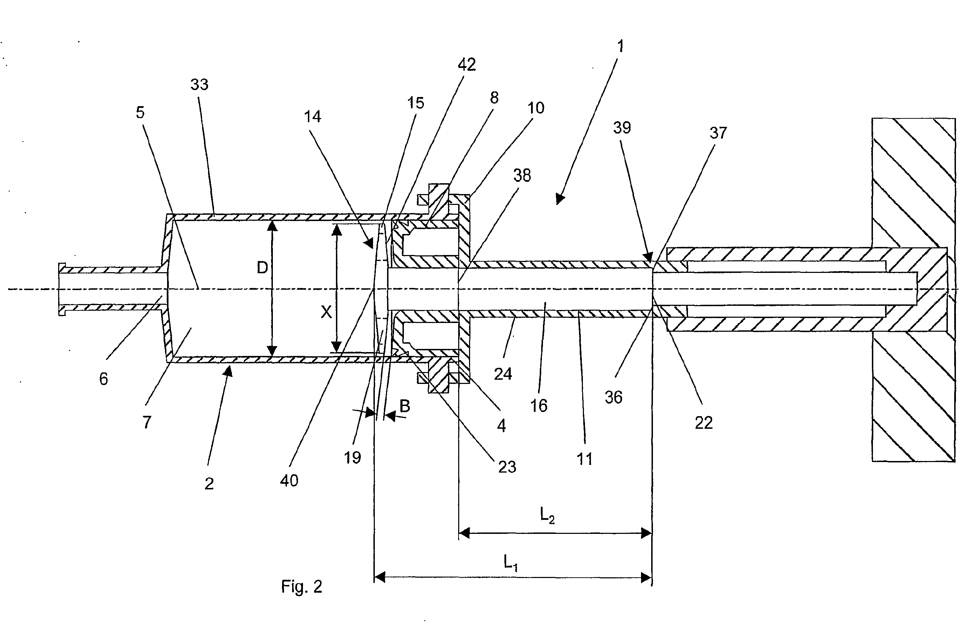

In Fig. 2 sind das Mischerwerkzeug 15 und der Kolben 8 in ihrer hintersten Position. Die zweiten Arretiermittel 39, welche die axiale Verschiebbarkeit des Mischers 14 gegen das hintere Ende 4 des Tubus 2 begrenzen, umfassen einen zweiten axialen Anschlag 22, welcher hier durch eine erste Querschnittskleinerung 36 der zur Längsachse 5 orthogonalen Querschnittsfläche des Mischerschaftes 16 und einer dazu komplementären zweiten Querschnittsverkleinerung 37 der zur Längsachse 5 orthogonalen Querschnittsfläche der Bohrung 11 in der Hülse 24 ausgestaltet ist. Durch diese zweiten Arretiermittel 39 ist erreichbar, dass, wenn das Mischwerkzeug 15 in seiner hintersten Position ist, zwischen der hinteren Stirnwand 42 des Mischwerkzeuges 15 und der vorderen Stirnfläche 23 des Kolbens 8 ein Minimalabstand B verbleibt. Dazu sind die Längen L₁ zwischen dem vorderen Ende 40 des Mischwerkzeuges 15 und der ersten Querschnittsverkleinerung 36 am Mischerschaft 16 und L₂ zwischen der gegen den Hohlraum 7 gerichteten Innenseite des Deckels 10 und der zweiten Querschnittsverkleinerung 37 in der Bohrung 11 in der Hülse 24 so bemessen, dass die Differenz der Längen L₁ - L₂ der Summe aus der axialen Abmessung des Mischwerkzeuges 15, der Länge des Kolbens 8 und dem Minimalabstand B entspricht. Ferner ist die senkrecht zur Längsachse 5 betrachtete maximale Abmessung X des Mischwerkzeuges 15 kleiner als der zur Längsachse 5 orthogonale Durchmesser D des Hohlraumes 7 im Tubus 2, so dass das Mischwerkzeug 15 auch von der seitlichen Hohlraumwand 33 beabstandet ist. Nach Beendung des Mischvorganges mittels des Mischers 14 kann der Mischerschaft 16 nach Lösen und Zurückziehen des Deckels 10 an einer Sollbruchstelle 38 abgebrochen werden, so dass das Dosieren des Zementes mittels des Kolbens 8 und einer dazu geeigneten Antriebsvorrichtung (nicht gezeichnet) durch den Mischerschaft 16, den Deckel 10 und die Antriebsmittel 17 nicht behindert wird. Als Antriebsvorrichtung kann beispielsweise ein handbetriebener, an den ersten Verriegelungsmitteln 29 lösbar befestigbarer Hebelantrieb, wie er in der EP 0 326 551 TEPIC offenbart wird, verwendet werden. Der gemischte Zement wird beim dosieren mittels des Kolbens 8 im Hohlraum 7 durch die Austrittsöffnung 6 gepresst. Dabei befindet sich das Mischwerkzeug 15 in einer zurückgezogenen, an die vordere Stirnfläche 23 des Kolbens 8 angrenzenden Position und wird vom Kolben 8 mitbewegt.

## Patentansprüche

1. Vorrichtung zur Lagerung und/oder Mischung und/oder Injektion von Zementen, insbesondere von Knochenzementen mit
A) einem Tubus (2), welcher eine Längsachse (5), ein vorderes Ende (3) mit einer Austrittsöffnung (6) und ein hinteres Ende (4) umfasst;
B) einem lösbar mit der Vorrichtung (1) verbindbaren Verschluss (12), wodurch die Austrittsöffnung (6) verschliessbar ist;
C) einem Deckel (10), welcher lösbar am hinteren Ende (4) des Tubus (2) befestigbar ist und koaxial mit einer Bohrung (11) durchbohrt ist;
D) einem im Hohlraum (7) axial und rotativ bewegbaren Mischer (14) mit einem Mischwerkzeug (15) und einem durch die Bohrung (11) in der Verschlusskappe (10) durchführbaren Mischerschaft (16); und
E) Antriebsmitteln (17) zur axialen und rotativen Bewegung des Mischers (14), welche ausserhalb des Tubus (2) mit dem Mischerschaft (16) verbindbar sind,
**dadurch gekennzeichnet, dass**
F) die Vorrichtung Arretiermittel (18;39) umfasst, welche die axiale Verschiebbarkeit des Mischerwerkzeuges (15) mindestens gegen das vordere Ende (3) des Tubus (2) begrenzen, so dass zwischen dem Mischwerkzeug (15) und der vorderen Hohlraumwand (20) ein Minimalabstand A verbleibt;
G) die ersten Arretiermittel (18) einen ersten, am Antriebsmittel (17) angebrachten axialen Anschlag (21) umfassen, welcher so ausgestaltet ist, dass die Antriebsmittel (17) am Deckel (10) zur Anlage kommen, wenn das Mischwerkzeug (15) in seiner vordersten Position ist; und
H) der Hohlraum (7) kreiszylindrisch ausgestaltet ist und senkrecht zur Längsachse (5) einen Durchmesser D aufweist und dass das Mischwerkzeug (15) senkrecht zur Längsachse (5) eine maximale Abmessung X aufweist, wobei X < D ist.

2. Vorrichtung nach Anspruch 1, **dadurch gekennzeichnet, dass** der Minimalabstand A zwischen 10 µm und 4000 µm beträgt.

3. Vorrichtung nach Anspruch 2, **dadurch gekennzeichnet, dass** der Minimalabstand A zwischen 25 µm und 1600 µm beträgt.

4. Vorrichtung nach einem der Ansprüche 1 bis 3, **dadurch gekennzeichnet, dass** die Vorrichtung (1)
a) zusätzlich einen Kolben (8) mit einer vorderen Stirnfläche (23) umfasst, welcher parallel zur Längsachse (5) im Hohlraum (7) verschiebbar ist und koaxial mit einer Durchgangsbohrung (9) durchbohrt ist; wobei
b) der Mischerschaft (16) durch die Durchgangsbohrung (9) im Kolben (8) und die Bohrung (11) im Deckel (10) durchführbar ist; und
c) dass die Vorrichtung zweite Arretiermittel (39) umfasst, welche einen zweiten, am Mischerschaft (16) und in der Bohrung (11) angebrachten axialen Anschlag (22) umfassen, welcher die axiale Verschiebbarkeit des Mischers (14) gegen das hintere Ende (4) des Tubus (2) begrenzt, so dass wenn sich der Kolben (8) und das Mischwerkzeug (15) in ihrer hintersten Position befindenden, zwischen dem Mischwerkzeug (15) und der vorderen Stirnfläche (23) des Kolbens (8) ein Minimalabstand B besteht.

5. Vorrichtung nach Anspruch 4, **dadurch gekennzeichnet, dass** der Minimalabstand B zwischen 10 µm und 4000 µm beträgt.

6. Vorrichtung nach Anspruch 5, **dadurch gekennzeichnet, dass** der Minimalabstand B zwischen 25 µm und 1600 µm beträgt.

7. Vorrichtung nach einem der Ansprüche 1 bis 6, **dadurch gekennzeichnet, dass** das Mischwerkzeug (15) ein vorderes Ende (40) aufweist und dass die Antriebsmittel (17) einen hülsenförmigen Fortsatz (26) mit einem vorderen, gegen den Deckel (10) gerichteten Ende (27) umfassen, wobei das vordere Ende (27) des Fortsatzes (26) vom vorderen Ende (40) des Mischwerkzeuges (15) parallel zur Längsachse (5) um eine Länge L beabstandet ist, und dass die Länge L so bemessen ist, dass wenn sich das Mischwerkzeug (15) in seiner vordersten Position befindet, das vordere Ende (27) an der Verschlusskappe (10) zur Anlage kommt.

8. Vorrichtung nach einem der Ansprüche 4 bis 7, **dadurch gekennzeichnet, dass** der Deckel (10) parallel zur Längsachse (5) eine sich gegen die Antriebsmittel (17) erstreckende, von der Bohrung (11) axial durchdrungene Hülse (24) umfasst und dass der zweite axiale Anschlag (22) durch eine Verkleinerung der zur Längsachse (5) orthogonalen Querschnittsfläche des Mischerschaftes (16) und einer dazu komplementären Verkleinerung der zur Längsachse (5) orthogonalen Querschnittsfläche der Bohrung (11) ausgeführt ist.

9. Vorrichtung nach einem der Ansprüche 1 bis 8, **dadurch gekennzeichnet, dass** die Differenz Δ zwischen dem Durchmesser D und der Abmessung ×, Δ = D - X zwischen 20 µm und 8000 µm beträgt.

10. Vorrichtung nach Anspruch 9, **dadurch gekennzeichnet, dass** die Differenz Δ zwischen dem Durchmesser D und der Abmessung X, Δ = D - X zwischen 50 µm und 3200 µm beträgt.

11. Vorrichtung nach einem der Ansprüche 1 bis 10, **dadurch gekennzeichnet, dass** der Deckel (10) mittels eines Bajonettverschlusses lösbar mit dem Tubus (2) verbindbar ist.

12. Vorrichtung nach Anspruch 11, **dadurch gekennzeichnet, dass** der Kolben (8) mittels einer Antriebsvorrichtung bewegbar ist, welche mittels dem Bajonettverschluss lösbar mit dem Tubus (2) verbindbar ist.

13. Vorrichtung nach einem der Ansprüche 1 bis 12, **dadurch gekennzeichnet, dass** das vordere Ende (3) des Tubus (2) einen hülsenförmigen, koaxial angeordneten und von der Austrittsöffnung (6) koaxial durchdrungenen Ansatz (31) umfasst und dass der Ansatz (12) eine Membran (13) zum Verschliessen der Austrittsöffnung (6) umfasst.

14. Vorrichtung nach einem der Ansprüche 4 bis 13; **dadurch gekennzeichnet, dass** der Kolben (8) eine zur Längsachse (5) konzentrische Dichtlippe (34) umfasst.

## Claims

1. Device for storing and/or mixing and/or injecting cements, especially bone cements with
A) a tube (2), which comprises a longitudinal axis (5), a front end (3) with an outlet opening (6) and a rear and (4),
B) a locking mechanism (12), which can be connected detachably with the device (1) and by means of which the outlet opening (6) can be closed off,
C) a lid (10), which can be fastened detachably at the rear end (4) of the tube (2) and which is traversed coaxially by a borehole (11),
D) a mixer (14), which can be moved axially and rotationally in the cavity (7), with a mixing tool (15) and a mixing shaft (16), which can be passed through the borehole (11) in the cover cap (10) and
E) driving means (17) for the axial and rotational movement of the mixer (14), which can be connected outside of the tube (2) with the mixer shaft (16),
**characterized in that**
F) the device comprises restraining means (18; 39), which form the boundary of the axial displaceability of the mixing tool (15) at least against the front end (3) of the tube (2), so that a minimum distance A remains between the mixing tool (15) and the front wall (20) of the cavity;
G) the first restraining means (18) comprise a first axial stop (21), which is mounted at the driving means (17), this first axial stop (21) being constructed so that the driving means (17) come at rest at the lid (10) when the mixing tool (15) is in its position furthest to the front; and
H) the cavity (7) is configured cylindrically and has a diameter D perpendicularly to the longitudinal axis (5) and that the mixing tool (15) has a maximum dimension X perpendicularly to the longitudinal axis (5), X being less than D.

2. The device of claim 1, **characterized in that** the minimum distance A is between 10 µm and 4000 µm.

3. The device of claim 2, **characterized in that** the minimum distance A is between 25 µm and 1600 µm.

4. The device of one of the claims 1 to 3, **characterized in that**
a) the device additionally comprises a piston (8) with a front face surface (23), which can be shifted parallel to the longitudinal axis (5) in the cavity (7) and is traversed axially by a through hole (9),
b) the mixer shaft (16) can be passed through the through hole (9) in the piston (8) and through the borehole (11) in the lid (10) and
c) the device comprises second restraining means (39) which comprise a second axial stop (22), which is mounted at the mixer shaft (16) and in the borehole (11) and limits the axial displaceability of the mixer (14) in the direction of the rear end (4) of the tube (2), so that, when the piston (8) and the mixing tool (15) are in their position furthest to the rear, there is a minimum distance B between the mixing tool (15) and the front face surface (23) of the piston (8).

5. The device of claim 4, **characterized in that** the minimum distance B is between 10 µm and 4000 µm.

6. The device of claim 5, **characterized in that** the minimum distance B is between 25 µm and 1600 µm.

7. The device of one of the claims 1 to 6, **characterized in that** the mixing tool (15) has a front end (40) and that the driving means (17) comprise a tubular continuation (26) with a front end (27) directed against the lid (10), the front end (27) of the continuation (26) being at a distance L, parallel to the longitudinal axis (5), from the front end (40) of the mixing tool (15), and that the length L is dimensioned so that the front end (27) comes to rest at the cover cap (10) when the mixing tool (15) is in its position furthest to the front.

8. The device of one of the claims 4 to 7, **characterized in that** the lid (10), parallel to the longitudinal axis (5), comprises a tube (24), which extends towards the driving means (17) and is traversed axially by the borehole (11), and that the second axial stop (22) is formed by a reduction in size of the cross-sectional surface of the mixer shaft (16), which is orthogonal to the longitudinal axis (5), and a complementary reduction in size of the cross-sectional surface of the borehole (11), which is orthogonal to the longitudinal axis (5).

9. The device of claim 1 to 8, **characterized in that** the difference Δ between the diameter D and the dimension X, Δ = D - X, is between 20 µm and 8000 µm.

10. The device of claim 9, **characterized in that** the difference Δ between the diameter D and the dimension X, Δ = D - X, is between 50 µm and 3200 µm.

11. The device of one of the claims 1 to 10, **characterized in that** the lid can be connected detachably with the tube (2) by means of a bayonet catch.

12. The device of claim 11, **characterized in that** the piston (8) can be moved by means of a driving device, which can be connected detachably with the tube (2) by means of the bayonet catch.

13. The device of one of the claims 1 to 12, **characterized in that** the front end (3) of the tube (2) comprises a tubular attachment (31), which is disposed coaxially and traversed coaxially by the outlet opening (6) and that the attachment (12) comprises a membrane (13) for closing off the outlet opening (6).

14. The device of one of the claims 4 to 13, **characterized in that** the piston (8) comprises a sealing lip (34), which is concentric with the longitudinal axis (5).

## Revendications

1. Dispositif de stockage et/ou de mélange et/ou d'injection de ciments, en particulier de ciments osseux, avec
A) un tube (2), lequel comprend un axe longitudinal (5), une extrémité avant (3) avec un orifice de sortie (6) et une extrémité arrière (4) ;
B) une fermeture (12) pouvant être couplée de manière amovible au dispositif (1), refermant ainsi l'orifice de sortie (6) ;
C) un capuchon (10), lequel peut être disposé de manière amovible sur l'extrémité arrière (4) du tube (2) et est traversé coaxialement par un alésage (11) ;
D) un mélangeur (14) mobile dans le sens axial et rotatif dans l'espace creux (7), avec un instrument de mélange (15) et une tige de mélangeur (16) pouvant être guidée dans l'alésage (11) dans le capuchon (10) ; et
E) des moyens d'entraînement (17) pour le déplacement axial et rotatif du mélangeur (14), lesquels peuvent en outre être reliés hors du tube (2) à la tige de mélangeur (16),
**caractérisé en ce que**
F) le dispositif comprend des moyens d'arrêt (18 ; 39), lesquels limitent la capacité de déplacement axial de l'instrument de mélange (15) au moins contre l'extrémité avant (3) du tube (2), de sorte qu'il reste une distance minimale A entre l'instrument de mélange (15) et la paroi avant de l'espace creux (20) ;
G) les premiers moyens d'arrêt (18) comprennent une première butée axiale (21) disposée au niveau des moyens d'entraînement (17), laquelle est conçue de telle manière que les moyens d'entraînement (17) viennent s'appuyer contre le capuchon (10) lorsque l'instrument de mélange (15) est dans sa position la plus avancée ; et
H) l'espace creux (7) est conçu sous forme de cylindre et présente, perpendiculairement à l'axe longitudinal (5), un diamètre D, et **en ce que** l'instrument de mélange (15) présente, perpendiculairement à l'axe longitudinal (5), une dimension maximale X, dans lequel X < D.

2. Dispositif selon la revendication 1, **caractérisé en ce que** la distance minimale A est comprise entre 10 *µ*m et 4000 *µ*m.

3. Dispositif selon la revendication 2, **caractérisé en ce que** la distance minimale A est comprise entre 25 *µ*m et 1600 *µ*m.

4. Dispositif selon l'une quelconque des revendications 1 à 3, **caractérisé en ce que** le dispositif (1)
a) comprend en outre un piston (8) avec une face avant (23), lequel peut être déplacé parallèlement à l'axe longitudinal (5) dans l'espace creux (7) et est traversé coaxialement par un trou de passage (9) ; dans lequel
b) la tige de mélangeur (16) peut être guidée dans le trou de passage (9) à l'intérieur du piston (8) et dans l'alésage (11) à l'intérieur du capuchon (10) ; et
c) le dispositif comprend des deuxièmes moyens d'arrêt (39), lesquels comprennent une deuxième butée axiale (22) disposée au niveau de la tige de mélangeur (16) et dans l'alésage (11), laquelle limite la capacité de déplacement axial du mélangeur (14) contre l'extrémité arrière (4) du tube (2), de sorte que lorsque le piston (8) et l'instrument de mélange (15) se trouvent dans leur position la plus reculée, il existe une distance minimale B entre l'instrument de mélange (15) et la face avant (23) du piston (8).

5. Dispositif selon la revendication 4, **caractérisé en ce que** la distance minimale B est comprise entre 10 *µ*m et 4000 *µ*m.

6. Dispositif selon la revendication 5, **caractérisé en ce que** la distance minimale B est comprise entre 25 µm et 1600 µm.

7. Dispositif selon l'une quelconque des revendications 1 à 6, **caractérisé en ce que** l'instrument de mélange (15) présente une extrémité avant (40), et **en ce que** les moyens d'entraînement (17) comprennent un prolongement en forme de manchon (26) avec une extrémité avant (27) dirigée vers le capuchon (10), dans lequel l'extrémité avant (27) du prolongement (26) est éloignée de l'extrémité avant (40) de l'instrument de mélange (15), parallèlement à l'axe longitudinal (5), selon une longueur L, et **en ce que** la longueur L est dimensionnée de telle manière que lorsque l'instrument de mélange (15) se trouve dans sa position la plus avancée, l'extrémité avant (27) vient s'appuyer sur le capuchon (10).

8. Dispositif selon l'une quelconque des revendications 4 à 7, **caractérisé en ce que** le capuchon (10) comprend, parallèlement à l'axe longitudinal (5), un manchon (24), s'étendant vers les moyens d'entraînement (17), traversé axialement par l'alésage (11), et **en ce que** la deuxième butée axiale (22) est réalisée par un rétrécissement de la surface de la section transversale, orthogonale à l'axe longitudinal (5), de la tige de mélangeur (16) et par un rétrécissement, complémentaire de celui-ci, de la surface de la section transversale, orthogonale à l'axe longitudinal (5), de l'alésage (11).

9. Dispositif selon l'une quelconque des revendications 1 à 8, **caractérisé en ce que** la différence Δ entre le diamètre D et la dimension ×, Δ = D - X, est comprise entre 20 *µ*m et 8000 *µ*m.

10. Dispositif selon la revendication 9, **caractérisé en ce que** la différence Δ entre le diamètre D et la dimension X, Δ = D - X, est comprise entre 50 *µ*m et 3200 *µ*m.

11. Dispositif selon l'une quelconque des revendications 1 à 10, **caractérisé en ce que** le capuchon (10) peut être couplé au tube (2) de manière amovible au moyen d'une fermeture à baïonnette.

12. Dispositif selon la revendication 11, **caractérisé en ce que** le piston (8) peut être déplacé au moyen d'un dispositif d'entraînement, lequel peut être couplé au tube (2) de manière amovible au moyen de la fermeture à baïonnette.

13. Dispositif selon l'une quelconque des revendications 1 à 12, **caractérisé en ce que** l'extrémité avant (3) du tube (2) comprend un embout (31) en forme de manchon, disposé coaxialement et traversé par l'orifice de sortie (6), et **en ce que** la fermeture (12) comprend une membrane (13) pour la fermeture de l'orifice de sortie (6).

14. Dispositif selon l'une quelconque des revendications 4 à 13, **caractérisé en ce que** le piston (8) comprend une lèvre d'étanchéité (34) concentrique à l'axe longitudinal (5).
